# DEMANDE DE BREVET EUROPEEN

(11) **EP 2 923 743 A1**
(43) Date de publication de la demande: **30.09.2015**
(21) Numéro de dépôt: 15160617.5
(22) Date de dépôt: 24.03.2015
(51) Int. Cl.: A63F 13/30, G01S 19/42, G06T 19/00

(54) **Dispositif de génération d'un flux video enrichi**

(30) Priorité: 26.03.2014 FR 1452554
(71) Demandeur: OPS 3S, 95220 Herblay (FR)
(72) Inventeur: Raffray, Yannick, 95220 HERBLAY (FR)
(74) Mandataire: Ipside

(57) **Abrégé**

La présente invention se rapporte à un dispositif (10) de génération d'un flux vidéo enrichi (33) comportant des moyens (12, 13) de capture d'un flux vidéo (29) géo-référencé d'une zone (14) dans laquelle évolue au moins un sujet d'intérêt (20-21), chaque sujet d'intérêt (20-21) étant muni d'au moins un capteur (15-16) et de moyens de radiocommunication (27), et des moyens d'interprétation des mesures dudit capteur (15-16) sous la forme de données structurées, des moyens de détermination d'une position) dudit sujet d'intérêt (20-21) au cours du temps dans le flux vidéo (29) géo-référencé, et des moyens de superposition des données structurées sur ledit flux vidéo (29) géo-référencé en fonction de ladite position déterminée au cours du temps.

## Description

### Domaine de l'invention

La présente invention concerne un dispositif de génération d'un flux vidéo enrichi. L'invention se rapporte à tous les domaines comportant des objets mobiles filmés dans leur environnement.

L'invention trouve une application particulièrement avantageuse pour le suivi des sports collectifs (handball, football...) et des sports individuels (tennis...), pour le suivi des entrainements militaires ainsi que pour enrichir des données de vidéo surveillance d'un site sensible.

### Etat de la technique

Après un match important, certains opérateurs de chaîne de télévision ont recours à des procédés de traitement vidéo pour enrichir les enregistrements des matchs. Par exemple, pour un match de football, il est possible d'enrichir un flux vidéo du match avec des indications sur le déplacement des joueurs pour expliquer le positionnement de la défense.

Pour les applications militaires, il est connu d'utiliser des balises satellites portées par tous les protagonistes afin de représenter en temps réel, sur un écran de contrôle, la position des troupes lors d'une simulation de combat. Cette simulation permet d'analyser les choix tactiques qui sont opérés par les différentes factions.

Dans une application de vidéo surveillance, il est connu de centraliser plusieurs types d'informations issues d'un site sensible. Ces informations peuvent être issues de caméras déployées et provenir de capteurs divers associés à des acteurs mobiles : camion, engin ou personnel.

Il est également connu d'utiliser ces deux approches simultanément. D'une part, un premier écran diffuse un flux vidéo d'une zone dans laquelle évoluent des sujets d'intérêt tels que des joueurs, des militaires ou encore des véhicules. D'autre part, chaque sujet d'intérêt est muni d'un ensemble de capteurs et de moyens de radiocommunication. Les données issues de l'ensemble de capteurs sont transmises à une unité de traitement et interprétées sous la forme de données structurées permettant de décrire la vitesse ou la position de chaque sujet d'intérêt. Ces données structurées sont alors représentées sur un second écran de visualisation.

### Exposé de l'invention

La présente invention vise à améliorer les dispositifs existants en intégrant les données structurées directement dans le flux vidéo.

A cet effet, l'invention concerne un dispositif de génération d'un flux vidéo enrichi comportant des moyens de capture d'un flux vidéo géo-référencé d'une zone dans laquelle évolue au moins un sujet d'intérêt, chaque sujet d'intérêt étant muni d'au moins un capteur et de moyens de radiocommunication, des moyens d'interprétation des mesures dudit capteur sous la forme de données structurées, des moyens de détermination d'une position dudit sujet d'intérêt au cours du temps dans le flux vidéo géo-référencé, et des moyens de superposition des données structurées sur ledit flux vidéo géo-référencé en fonction de ladite position déterminée au cours du temps.

L'invention permet ainsi de visualiser, de manière unifiée, les données structurées directement dans ledit flux vidéo sur le même écran de visualisation et à la position même des objets mobiles dans le flux vidéo. L'invention permet ainsi d'améliorer la qualité d'analyse des situations de jeux pour un sport collectif, des situations d'engagement pour une simulation militaire, ou des situations d'évaluation des risques pour un site sensible. Dans le cas d'un site sensible, le personnel peut être équipé d'un transpondeur assurant à la fois des besoins de sécurisation et d'authentification.

Selon un mode de réalisation, l'au moins un capteur de chaque sujet d'intérêt étant un récepteur mobile de positionnement par satellite, les moyens de détermination de ladite position comportent un récepteur fixe de positionnement par satellite associé à une position connue, ledit récepteur fixe permettant de corriger ladite position déterminée de chaque récepteur mobile en fonction de l'écart entre la position estimée du récepteur fixe et sa position connue. Ce mode de réalisation permet d'obtenir une position de chaque sujet d'intérêt dans une zone extérieure particulièrement étendue. La précision nominale typique est de moins de 1 m sur trois dimensions et de 4 cm si le sujet d'intérêt est immobile au moins 5 min.

Selon un mode de réalisation, l'au moins un capteur de chaque sujet d'intérêt étant une puce mobile, les moyens de radiocommunication comportent des moyens de détermination de temps de vol entre ladite puce mobile et au moins deux stations fixes interconnectées, ladite position étant déterminée en fonction desdits temps de vol. Ce mode de réalisation permet d'obtenir une position de chaque sujet d'intérêt dans une zone à l'intérieur d'un bâtiment. La précision nominale typique est de moins de 10 cm sur trois dimensions. De préférence, les stations fixes sont interconnectées par un réseau sans fil, par exemple de type WiFi (pour « Wireless Fidelity » dans la littérature anglo-saxonne), 3G (pour 3^{ème} génération) ou 4G/LTE (pour 4^{ème} génération). Ladite puce mobile peut être intégrée dans un transpondeur autonome en énergie ou au sein d'un Smartphone.

Selon un mode de réalisation, la superposition des données structurées sur le flux vidéo géo-référencé est effectuée en temps réel. Ce mode de réalisation permet d'effectuer une analyse en temps réel des situations de jeux pour un sport collectif ou des situations d'engagement pour une simulation militaire. Par exemple, lors d'un match de Handball, le spectateur peut suivre sur un écran différentes informations relatives aux joueurs dans une bulle qui leur sont associés et qui les suivent lors de leurs déplacements. Pour un autre exemple, pour la sécurisation d'un site sensible, une centrale de contrôle peut calculer les risques liés aux mouvements du personnel afin de détecter l'accès à une zone de pénétration interdite ou de limiter la température ou la toxicité d'une pièce.

Selon un mode de réalisation, l'au moins un capteur de chaque sujet d'intérêt étant une centrale inertielle, le dispositif comporte des moyens aptes à intégrer les mouvements dudit sujet d'intérêt pour estimer son orientation, sa vitesse linéaire et sa position.

Selon un mode de réalisation, ladite centrale inertielle comporte trois gyromètres, trois accéléromètres, un baromètre et un magnétomètre.

Selon un mode de réalisation, l'au moins un capteur de chaque sujet d'intérêt étant une ceinture pectorale, le dispositif comporte des moyens aptes à intégrer les mesures dudit sujet d'intérêt pour estimer son niveau de stress ou ses émotions. Ce mode de réalisation permet d'obtenir des informations particulièrement pertinentes sur chaque sujet d'intérêt telles que les mesures en continu de l'activité du coeur et de la fréquence respiratoire, les mesures dudit sujet d'intérêt pour estimer son taux d'humidité ou son taux de sudation ou les mesures en continu de la température et de la conductance de la peau.

Selon un mode de réalisation, l'au moins un capteur de chaque sujet d'intérêt est intégré dans un bracelet.

Selon un mode de réalisation, l'au moins un capteur de chaque sujet d'intérêt est intégré dans les fibres d'un tissu intelligent.

Selon un mode de réalisation, l'au moins un capteur de chaque sujet d'intérêt est une étiquette collée sur la peau dudit sujet d'intérêt.

### Brève description des dessins

On comprendra mieux l'invention à l'aide de la description, faite ci-après à titre purement explicatif, des modes de réalisation de l'invention, en référence aux Figures dans lesquelles :
- la Figure 1 illustre un dispositif de génération d'un flux vidéo enrichi selon un premier mode de réalisation de l'invention ;
- la Figure 2 illustre les étapes de traitement du dispositif de la Figure 1 ; et
- la Figure 3 illustre un flux vidéo enrichi selon un deuxième mode de réalisation de l'invention.

### Description détaillée des modes de réalisation de l'invention

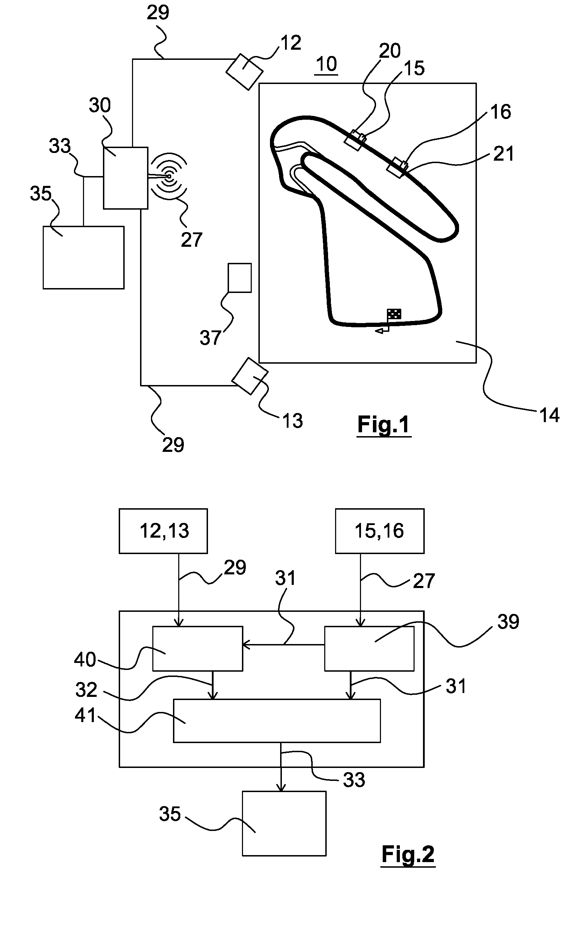

Les Figures 1 et 2 illustrent un dispositif 10 de génération d'un flux vidéo enrichi 33 pour le suivi d'une course automobile entre deux sujets d'intérêt 20-21. Une zone 14 dans laquelle évoluent les sujets d'intérêt 20-21 est filmée par deux moyens 12-13 de capture d'un flux vidéo 29 géo-référencé. Dans ce mode de réalisation, les sujets d'intérêt 20-21 sont les pilotes ou les véhicules mais en variante, les sujets d'intérêt 20-21 peuvent correspondre à des joueurs ou des militaires.

Chaque sujet d'intérêt 20-21 est équipé d'au moins un capteur 15-16 et de moyens de radiocommunication 27. Les moyens de radiocommunication 27 sont couplés à une unité de traitement 30 recevant également le flux vidéo 29 géo-référencé en provenance des moyens 12-13 de capture. La Figure 2 illustre particulièrement le traitement effectué par l'unité de traitement 30.

Les mesures issues des capteurs 15-16 sont transmises à des moyens d'interprétation 39 afin d'obtenir des données structurées 31. Ces données structurées 31 décrivent des informations pertinentes sur chaque sujet d'intérêt 15-16 telles que la vitesse ou la position de chaque sujet d'intérêt 15-16. La mesure de la position de chaque sujet d'intérêt 15-16 peut être effectuée au moyen d'un récepteur mobile de positionnement par satellite et corrigée par la mesure de la position d'un récepteur fixe 37 dont la position est connue.

Le flux vidéo 29 géo-référencé est traité par des moyens de détermination 40 à l'aide des données structurées 31 donnant la position de chaque sujet d'intérêt 15-16 dans le flux vidéo 29 au cours du temps. A cet effet, le flux vidéo 29 est géo-référencé, c'est-à-dire que la position des moyens 12-13 de capture et/ou des éléments fixes contenus dans le flux vidéo 29 est connue. Dans le cas des Figures 1 et 2, les éléments fixes géo-référencés du flux vidéo 29 peuvent correspondre aux lignes du circuit ou à des repères spécifiques posés sur le circuit. En connaissant la position des éléments fixes du flux vidéo 29 et la position des sujets d'intérêt 15-16 au cours du temps, il est possible d'estimer la position des sujets d'intérêt 15-16 dans le flux vidéo 29 géo-référencé.

Les données structurées 31 sont également transmises à des moyens de superposition 41 aptes à intégrer les données structurées 31 sur le flux vidéo 29 géo-référencé afin de former le flux vidéo enrichi 33. De préférence, ce flux vidéo enrichi 33 est diffusé sur un écran de visualisation 35 afin que les spectateurs ou les superviseurs des deux pilotes puissent analyser la course. En variante, le flux vidéo enrichi 33 peut être diffusé sur un autre média tel qu'une tablette numérique, un Smartphone ou des lunettes de réalité augmentée. En outre, certains médias peuvent afficher seulement certaines informations. Par exemple, les téléspectateurs peuvent obtenir le flux vidéo enrichi 33 directement sur leurs téléviseurs alors que les spectateurs présents autour du circuit peuvent obtenir uniquement les données structurées sur une tablette tactile, un Smartphone ou des lunettes de réalité augmentée.

De préférence, les traitements sont effectués en temps réel, c'est-à-dire dans un temps déterminé.

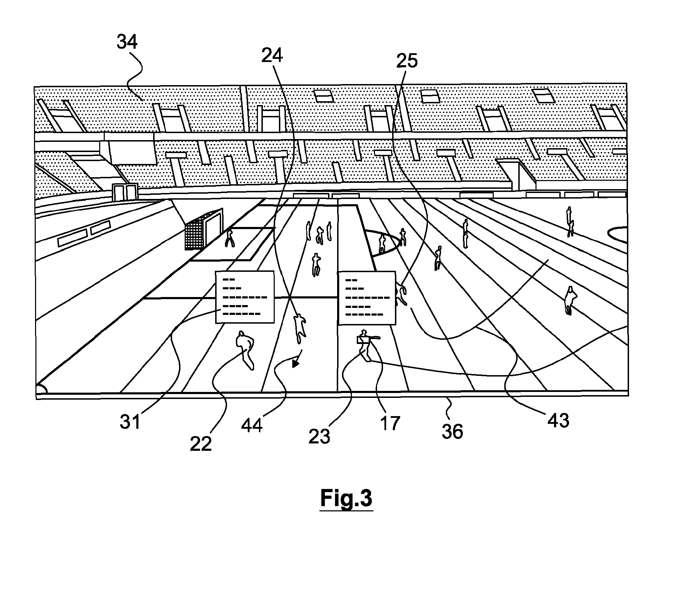

Le second mode de réalisation de la Figure 3 illustre un écran de visualisation 36 diffusant un flux vidéo enrichi 34 d'un match de football. Les joueurs correspondant aux sujets d'intérêt 22-25 sont munis de capteurs 17 disposés sur une ceinture pectorale. En variante, les capteurs peuvent être intégrés dans un bracelet, dans les fibres d'un tissu intelligent ou dans une étiquette collée sur la peau. Les capteurs 17 peuvent correspondre à des moyens de mesure en continu de l'activité du coeur, de la fréquence respiratoire, de la température de la peau ou de la conductance de la peau. Ces différents capteurs permettent aux moyens d'interprétation 39 d'estimer le niveau de stress, les émotions, le taux d'humidité ou de sudation de chaque sujet d'intérêt 22-25.

Les capteurs 17 peuvent également comprendre une centrale inertielle comprenant trois gyromètres, trois accéléromètres, un baromètre et un magnétomètre aptes à intégrer les mouvements des sujets d'intérêt 22-25 pour estimer leurs orientations ou leurs vitesses linéaires. La position de chaque sujet d'intérêt 22-25 est également calculée à l'aide d'une puce mobile apte à déterminer des temps de vol entre la puce mobile et plusieurs stations fixes (non représentées) disposées autour du stade et interconnectées entre elles.

Ces différentes données structurées sont incrustées sur le flux vidéo au dessus de chaque joueur. Dans l'exemple de la Figure 3, les données structurées associées à chaque joueur correspondent au numéro du joueur, à son nom, à sa vitesse, à son accélération et au nombre de kilomètres parcourus durant la partie. En outre, les moyens d'interprétation 39 sont également capables de déterminer des courses 43 ou des trajectoires 44. Ces informations permettent ainsi d'améliorer les stratégies d'attaque ou de remplacement au cours de la partie.

L'invention permet ainsi d'intégrer des données structurelles 31 sur un flux vidéo 29 géo-référencé au niveau des sujets d'intérêt 20-25. Cette incorporation est particulièrement utile pour analyser en temps réel des situations de combat dans une simulation militaire ou pour un évènement sportif.

## Revendications

1. Dispositif (10) de génération d'un flux vidéo enrichi (33, 34), **caractérisé en ce qu'**il comporte :
- des moyens (12, 13) de capture d'un flux vidéo (29) géo-référencé d'une zone (14) dans laquelle évolue au moins un sujet d'intérêt (20-25), chaque sujet d'intérêt (20-25) étant muni d'au moins un capteur (15-17) et de moyens de radiocommunication (27), et
- des moyens d'interprétation (39) des mesures dudit capteur (15-17) sous la forme de données structurées (31),
**caractérisé en ce qu'**il comporte également :
- des moyens de détermination (40) d'une position (32) dudit sujet d'intérêt (20-25) au cours du temps dans le flux vidéo (29) géo-référencé, et
- des moyens de superposition (41) des données structurées (31) sur ledit flux vidéo (29) géo-référencé en fonction de ladite position (32) déterminée au cours du temps.

2. Dispositif selon la revendication 1, **caractérisé en ce que**, l'au moins un capteur (15-17) de chaque sujet d'intérêt (20-25) étant un récepteur mobile de positionnement par satellite, les moyens de détermination (40) de ladite position comportent un récepteur fixe (37) de positionnement par satellite associé à une position connue, ledit récepteur fixe (37) permettant de corriger ladite position (32) déterminée de chaque récepteur mobile en fonction de l'écart entre la position estimée du récepteur fixe (37) et sa position connue.

3. Dispositif selon la revendication 1 ou 2, **caractérisé en ce que**, l'au moins un capteur (15-17) de chaque sujet d'intérêt (20-25) étant une puce mobile, les moyens de radiocommunication (27) comportent des moyens de détermination de temps de vol entre ladite puce mobile et au moins deux stations fixes interconnectées, ladite position (32) étant déterminée en fonction desdits temps de vol.

4. Dispositif selon l'une des revendications précédentes, **caractérisé en ce que** la superposition des données structurées (31) sur le flux vidéo (29) géo-référencé est effectuée en temps réel.

5. Dispositif selon l'une des revendications précédentes, **caractérisé en ce que**, l'au moins un capteur (15-17) de chaque sujet d'intérêt (20-25) étant une centrale inertielle, le dispositif comporte des moyens aptes à intégrer les mouvements dudit sujet d'intérêt (20-25) pour estimer son orientation, sa vitesse linéaire et sa position.

6. Dispositif selon la revendication 5, **caractérisé en ce que** ladite centrale inertielle comporte trois gyromètres, trois accéléromètres, un baromètre et un magnétomètre.

7. Dispositif selon l'une des revendications précédentes, **caractérisé en ce que**, l'au moins un capteur (15-17) de chaque sujet d'intérêt (20-25) étant une ceinture pectorale, le dispositif comporte des moyens aptes à intégrer les mesures dudit sujet d'intérêt pour estimer son niveau de stress ou ses émotions.

8. Dispositif selon l'une des revendications précédentes, **caractérisé en ce que**, l'au moins un capteur (15-17) de chaque sujet d'intérêt (20-25) est intégré dans un bracelet.

9. Dispositif selon l'une des revendications précédentes, **caractérisé en ce que**, l'au moins un capteur (15-17) de chaque sujet d'intérêt (20-25) est intégré dans les fibres d'un tissu intelligent.

10. Dispositif selon l'une des revendications précédentes, **caractérisé en ce que**, l'au moins un capteur (15-17) de chaque sujet d'intérêt (20-25) est une étiquette collée sur la peau dudit sujet d'intérêt (20-25).
